# EUROPÄISCHE PATENTSCHRIFT

(11) **EP 1 734 364 B1**
(45) Veröffentlichungstag und Bekanntmachung des Hinweises auf die Patenterteilung: **15.10.2014**
(21) Anmeldenummer: 06115660.0
(22) Anmeldetag: 19.06.2006
(51) Int. Cl.: G01N 33/28

(54) **Verfahren und Vorrichtung für eine beschleunigte Oxidationsbestimmung von Kraftstoffen oder Mineralölprodukten sowie ein Computerprogramm zur Steuerung einer solchen Vorrichtung und ein entsprechendes computerlesbares Speichermedium**
Method and device for accelerated determination of the oxidation of fuels or mineral oil products and a computer program for controlling such a device and a corresponding computer readable storage medium
Procédé et dispositif destinés à la détermination accélérée de l'oxydation de carburants ou de produits d'huile minérale, tout comme programme informatique destiné à la commande d'un tel dispositif et support de stockage pouvant être lu par un ordinateur correspondant

(30) Priorität: 17.06.2005 DE 102005028896
(43) Veröffentlichungstag der Anmeldung: 20.12.2006
(73) Patentinhaber: Anton Paar ProveTec GmbH, 15827 Blankenfelde-Mahlow (DE)
(72) Erfinder: Handschuck, Bernhard, 12305 Berlin (DE); Heine, Christian, 13125 Berlin (DE); Neumann, Andrea, 15366 Berlin (DE); Schulz, Rüdiger, 12249 Berlin (DE); Wierzbicki, Volkmar, 12309 Berlin (DE)
(74) Vertreter: Gulde & Partner

(56) Entgegenhaltungen:
- DE-A1- 3 321 015
- DE-A1- 19 746 723
- US-A- 4 456 539
- CERNY J, ZELINKA M: "Oxidation Stability of Lubricants Measured by a PDSC Technique" PETROLEUM AND COAL, Bd. 46, Nr. 3, 2004, Seiten 56-62, XP009086924 ISSN: 1335-3055

## Beschreibung

Die Erfindung betrifft ein Verfahren und eine Vorrichtung für eine beschleunigte Oxidationsbestimmung von Kraftstoffen oder Mineralölprodukten sowie ein Computerprogramm zur Steuerung einer solchen Vorrichtung und ein entsprechendes computerlesbares Speichermedium, welche insbesondere einsetzbar sind für die Simulation der Alterung von Kraftstoffen oder Mineralölprodukten.

Zur Bestimmung der Oxidationszeit z. B. von Kraftstoffen bestehen internationale Normen, wie z.B. DIN 51 780 (veraltet), (EN)ISO 7536 oder ASTM D 525. Diese Normen legen fest, dass eine 50 ml Kraftstoffprobe in ein Druckgefäß mit beschriebenen Abmaßen eingebracht, verschlossen und mit Sauerstoff auf 700 kPa aufgeladen wird, um den Zeitpunkt des später einsetzenden Druckabfalls bestimmen zu können, der das genormte Oxidationsergebnis darstellt.

Herkömmlicherweise ist eine Anordnung zur beschleunigten Oxidationsbestimmung von Kraftstoffen und Mineralölprodukten mit einem Druck-Messgerät versehen und wird in ein auf 100°C temperiertes Wasserbad gebracht. Unter diesen Bedingungen (erhöhte Temperatur, sowie Sauerstoff unter Druckeinfluss) oxidiert der Kraftstoff üblicherweise erheblich schneller, als bei normaler Lagerung, was Rückschlüsse auf die unter normalen Umgebungsbedingungen diesbezüglich zu erwartende Lebensdauer des Kraftstoffes schließen lässt.

Die manuelle oder automatische Auswertung erfolgt aufgrund der nach einiger Zeit einsetzenden Oxidation, die normgemäß als Brechpunkt mit 14kPa Druckabfall in 15 Minuten definiert ist und bei automatischer Erkennung zum Ende der Kurven-/ Datenaufzeichnung führt.

Bei heutigen Kraftstoffen liegen die Brechpunkte unter den o.a. Testbedingungen typisch bei 6...8 Stunden, was als sehr nachteilig empfunden werden muss, wenn man diese Zeiten mit denen anderer Laboruntersuchungen zur Lieferfreigabe vergleicht, die meist im Minutenbereich abgeschlossen sind. Weiterhin gibt es Kraftstoffe, deren Additive die Oxidation derart verlangsamen, dass die in der Norm ASTM D 525 beschriebene Rampensteilheit für das gesuchte Ergebnis nicht erreicht wird.

Eine Vorrichtung zur Durchführung einer solchen Bestimmung der Oxidationsbeständigkeit von Kraftstoffen wird in der Veröffentlichung DE 197 46 723 A1 beschrieben. Insbesondere wird eine in die Vorrichtung eingebrachte Probe auf eine Temperatur von 100°C erhitzt.

Als weitere Verfahren werden thermische Verfahren eingesetzt, wie beispielsweise die dynamische Wärmestrom- oder dynamische Differenz-Kalorimetrie (Differential Scanning Calorimetry DSC) bzw. die Druck-Differenz Kalorimetrie (PDSC), bei denen der Wärmestrom einer kleinen Probemenge unter definierten Messbedingungen ermittelt wird. Dabei zeigt ein örtlicher Temperaturanstieg in der Probe gegenüber der Umgebung eine Oxidation an.

In der Veröffentlichung 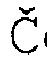 erný, Jaroslav, Miroslav Zelinka: Oxidation Stability of Lubricants Measured by a PDSC Technique, Petroleum & Coal, Bd. 46, Nr. 3, 2004, Seiten 56 bis 62 wird ein spezielles PDSC-Verfahren beschrieben.

Die Aufgabe der Erfindung besteht somit darin, ein Verfahren und eine Vorrichtung für eine beschleunigte Oxidationsbestimmung von Kraftstoffen oder Mineralölprodukten sowie ein Computerprogramm zur Steuerung einer solchen Vorrichtung und ein entsprechendes computerlesbares Speichermedium bereitzustellen, welche die oben genannten Mängel beheben und es insbesondere erlauben, die Zeiten für die Bestimmung des Brechpunktes zu reduzieren.

Diese Aufgabe wird erfindungsgemäß durch die Merkmale in den Ansprüchen 1, 6, 10 und 11 gelöst. Zweckmäßige Ausgestaltungen der Erfindung sind in den Unteransprüchen enthalten.

Ein Verfahren für eine beschleunigte Oxidationsbestimmung von Kraftstoffen oder Mineralölprodukten nach der Erfindung ist dadurch ausgezeichnet, dass eine Probe der Kraftstoffe oder Mineralölprodukte direkt in einen Druckbehälter eingebracht und die Probe durch zumindest ein im Druckbehälter und/oder in der Probe angeordnetes Mittel zur Temperaturveränderung auf eine gewünschte Temperatur gebracht und der Druck in dem Druckbehälter überwacht wird, um die Oxidation zu bestimmen. Erfindungsgemäß wird dabei eine Temperatur von über 100°C vorgegeben. Vorzugsweise wird die vorgebbare Temperatur während der Oxidationsbestimmung konstant gehalten. Die Oxidation des Kraftstoffs bzw. des Mineralölprodukts wird ermittelt, indem Brechpunktes zur Festlegung eines das Absinken des absoluten Druckwertes überwacht und der Brechpunkt als der Punkt festgesetzt wird, an dem der Druck um 10% von seinem Maximum abgesunken ist.

In einer bevorzugten Ausführungsform des erfindungsgemäßen Verfahrens ist vorgesehen, dass eine Probe von einem Volumen von 3 bis 20 ml eingebracht wird. Vorzugsweise wird ein Probevolumen von ca. 5 ml eingebracht.

In einer bevorzugten Ausführungsform des erfindungsgemäßen Verfahrens ist weiter vorgesehen, dass Druckbehälter und/oder Probe mit Ultraschall beaufschlagt werden.

Ein besonderer Vorteil der erfindungsgemäßen Vorrichtung für eine beschleunigte Oxidationsbestimmung von Kraftstoffen oder Mineralölprodukten besteht darin, dass eine sehr schnelle Temperaturänderung, insbesondere eine schnelle Temperaturerhöhung, bewirkt wird. Dies wird erreicht, indem die Vorrichtung gemäß Anspruch 6 einen Druckbehälter zur Aufnahme von Proben der Kraftstoffe oder Mineralölprodukte umfasst. In einer bevorzugten Ausführungsform werden kleine thermische Massen eingesetzt, wodurch der Vorgang weiter beschleunigt wird.

In einer bevorzugten Ausführungsform der erfindungsgemäßen Vorrichtung ist vorgesehen, dass das zumindest eine Mittel zur Temperaturveränderung als Heizung ausgebildet ist. Vorteilhafterweise wird eine Heizung mit Regelung eingesetzt. Dabei ist das zumindest eine Mittel zur Temperaturveränderung im Druckbehälter und/oder in der Probe angeordnet.

Erfindungsgemäß ist vorgesehen, dass die Innenwand des Druckbehälters wenigstens teilweise eine resistente Oberfläche aufweist. Beispielsweise kann der Druckbehälter aus Aluminium und die resistente Oberfläche aus Gold ausgeführt sein.

In einer weiteren bevorzugten Ausführungsform der erfindungsgemäßen Vorrichtung ist vorgesehen, dass am Boden des Druckbehälters und/oder in der Probe ein ultraschall-betriebener Schwinger angeordnet ist.

Zur Steuerung der erfindungsgemäßen Vorrichtung wird vorteilhafterweise ein Computerprogramm eingesetzt. Ein solches erfindungsgemäßes Computerprogramm gemäß Anspruch 10 für eine beschleunigte Oxidationsbestimmung ermöglicht es einer Datenverarbeitungseinrichtung, nachdem es in den Speicher der Datenverarbeitungseinrichtung geladen worden ist, ein Verfahren für eine beschleunigte Oxidationsbestimmung von Kraftstoffen oder Mineralölprodukten durchzuführen, wobei eine Probe eines Kraftstoff- oder Mineralölproduktes direkt in einen Druckbehälter eingebracht wird und die Probe durch zumindest ein im Druckbehälter und/oder in der Probe angeordnetes Mittel zur Temperaturveränderung auf eine vorgebbare Temperatur über 100°C gebracht und der Druck in dem Druckbehälter überwacht wird, um die Oxidation zu bestimmen, wobei zur Festlegung eines Brechpunktes das Absinken des absoluten Druckwertes überwacht und der Brechpunkt als der Punkt festgesetzt wird, an dem der Druck um 10% von seinem Maximum abgesunken ist.

Beim Vertrieb der Erfindung kann es von Vorteil sein, wenn derartige Computerprogramme (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren gemäß Anspruch 13 nutzbar gemacht werden, bei dem ein Computerprogramm nach Anspruch 10 aus einem elektronischen Datennetz wie beispielsweise aus dem Internet auf eine an das Datennetz angeschlossene Datenverarbeitungseinrichtung heruntergeladen wird.

Um das erfindungsgemäße Verfahren für eine beschleunigte Oxidationsbestimmung von Kraftstoffen oder Mineralölprodukten durchzuführen, ist vorgesehen, ein computerlesbares Speichermedium gemäß Anspruch 11 einzusetzen, auf dem ein Programm gespeichert ist, das es einer Datenverarbeitungseinrichtung ermöglicht, nachdem es in den Speicher der Datenverarbeitungseinrichtung geladen worden ist, ein Verfahren für eine beschleunigte Oxidationsbestimmung von Kraftstoffen oder Mineralölprodukten durchzuführen, wobei eine Probe eines Kraftstoff- oder Mineralölproduktes direkt in einen Druckbehälter eingebracht wird und die Probe durch zumindest ein im Druckbehälter und/oder in der Probe angeordnetes Mittel zur Temperaturveränderung auf eine vorgebbare Temperatur über 100°C gebracht und der Druck in dem Druckbehälter überwacht wird, um die Oxidation zu bestimmen, wobei zur Festlegung eines Brechpunktes das Absinken des absoluten Druckwertes überwacht und der Brechpunkt als der Punkt festgesetzt wird, an dem der Druck um 10% von seinem Maximum abgesunken ist.

Zusätzlich oder alternativ können computerlesbare Speichermedien gemäß Anspruch 12 bereitgestellt werden, auf denen ein Computerprogramm nach Anspruch 10 gespeichert ist. Die Erfindung weist folgende Vorteile auf:

### Beschleunigte Methode:

Die Erfindung schlägt vor, den genormten Test durch Temperaturerhöhung zu beschleunigen. Es wurde dabei erkannt, dass eine Temperaturerhöhung von 100°C auf 140°C die Oxidationszeit auf etwa 7% des Wertes sinken lässt.
Eine wichtige Voraussetzung hierfür ist eine schnellere Wärmeübertragung in die Probe. In der Standard-Methode wird vorgeschrieben, dass der Kraftstoff zunächst in ein Glasgefäß und diese Einheit dann in den Druckbehälter eingebracht werden soll, wodurch ein schneller Wärmefluss in das zu untersuchende Material behindert wird.

### Passive Oberfläche im Druckbehälter:

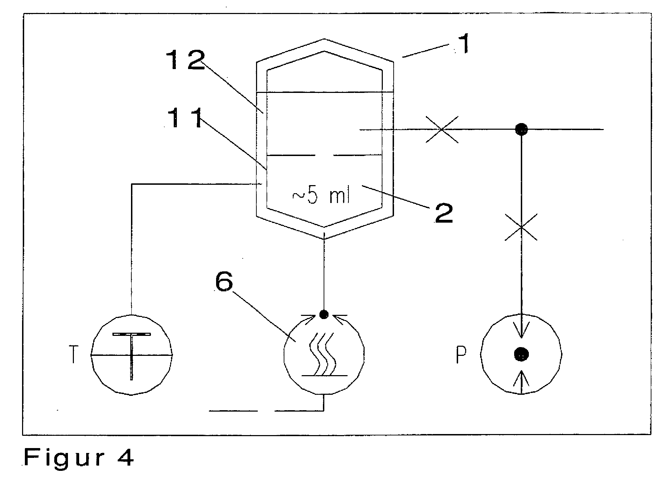

Daher ist ein Ansatz dieser beschleunigten Methode, den Kraftstoff direkt in den Druckbehälter - ohne Glasgefäß - einzubringen. Chemische/physikalische/katalytische Effekte werden durch eine resistente Oberfläche z.B. aus Gold (vgl. Figur 4) bei der nun unmittelbaren Berührung des Kraftstoffes mit dem Metall unterbunden.

### Temperier-Block:

Nach dem Verschließen des Behälters wird dieser nicht mehr in ein Flüssigkeitsbad gesetzt, sondern der Druckbehälter-Metallblock (nachfolgend kurz mit Block bezeichnet) mit der Probe wird über eine eingebaute temperaturgeregelte Heizung (vgl.

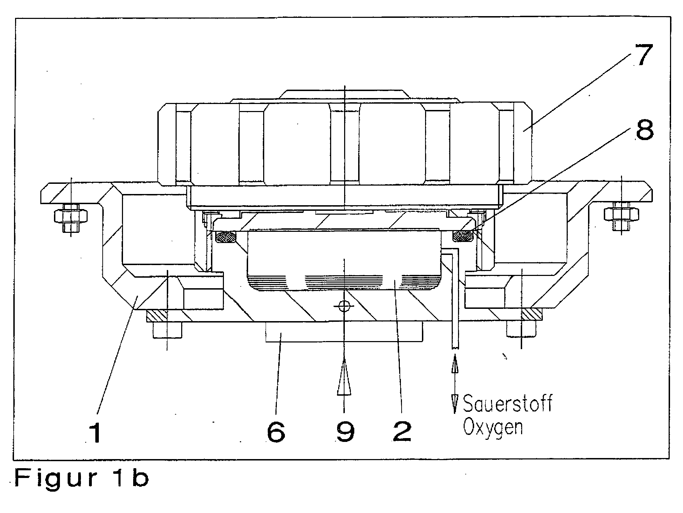

Figur 1b) schnell und genau auf die gewünschte Temperatur gebracht.
Der Fühler für diese Regelung sitzt im Block oder in der Kraftstoffprobe selbst. Dadurch ist ein gut reproduzierbares Temperaturverhalten erreicht.

### Geänderte Brechpunkt-Erfassung:

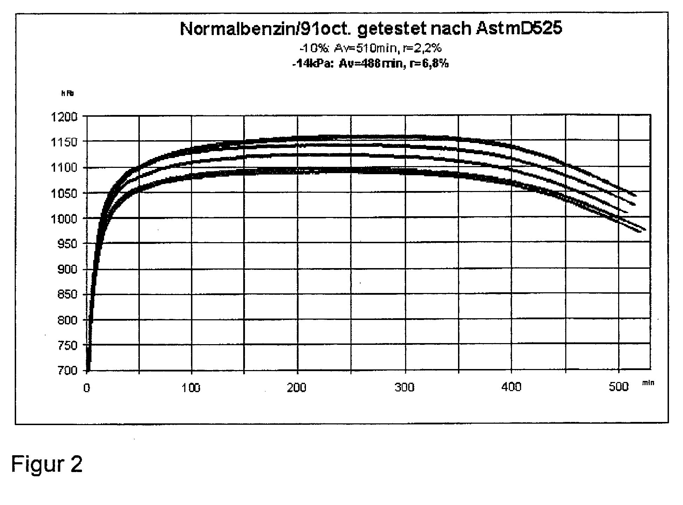

Kraftstoffe, die heute auf dem Markt sind, haben in der Regel eine zunehmend größere Lebensdauer hinsichtlich ihrer Oxidationsbeständigkeit. Hierdurch kann es passieren, dass aufgrund der damit verbundenen evtl. zu geringen Abfallrampe des Oxidationsdruckes keinen Brechpunkt nach herkömmlicher Definition erzeugt, obwohl der Kraftstoff mit Sauerstoff reagiert.
Ferner lässt sich aus den in Figur 2 dargestellten Kurven unschwer erkennen, dass auch bei gefundenem Testende diese Brechpunkte aufgrund des relativ flachen Druckabfalles nur ungenau bestimmbar sind.
Der Brechpunkt wird daher bei der beschleunigten Methode bestimmt, indem der absolute Druckwert überwacht wird. Er ist dann erreicht, wenn der Druck um 10% von seinem erreichten Maximum abgesunken ist - unabhängig von der Sink-Geschwindigkeit.
Aus Figur 2 ist zu ersehen, dass die Wiederholbarkeit "r" in der beschleunigten Methode mit 2,2% gegenüber 6,8% wesentlich besser abschneidet. Die Brechpunkt-Ergebnisse beider Methoden weichen im Mittelwert (Av) etwas voneinander ab.

Verbesserungen der beschleunigten Methode gegenüber der Standard-Methode:

| | Standard Methode | beschleunigte Methode |
|---|---|---|
| Reinigung des Druckbehälters | ja | ja |
| Einfüllen d. Probe 50/5 ml | 50 ml | 5 ml |
| Deckel des Druckbehälters schließen | ja | ja |
| mit Sauerstoff 1x spülen | ca. 3 min | automatisch |
| endgültig Sauerstoff einbringen | 1 min | automatisch |
| Anfangsdruck des eingebrachten Sauerstoffs | 700 kPa | 500 kPa |
| Heizstart im Flüssigbad/Heizblock | ca. 1 min (Bad) | aut. (Block) |
| konstante Oxidationstemperatur | 100°C | 140°C |
| Gaslecküberwachung | automatisch | automatisch |
| Erfassen des Oxidationsendes (aut.) | ca. <500 min | ca. <45 min |
| erf. Anwesenheit zum Abkühlen | ca. 1 min | automatisch |
| Langsames Ablassen des oxidierten Gases | 2 min | automatisch |
| | | |
| Totales Volumen des Druckbehälters | ca. 160 ml | ca. 20 ml |
| Probemenge | 50 ml | 5 ml |
| Ultraschallmischer möglich | nein | ja |

### Verbesserte Sicherheit:

Das Volumen des Blocks und der Probe (beschleunigte Methode) wurde in einer bevorzugten Ausführung etwa 10 x kleiner gewählt als das der Standard-Methode. Dieses hat zur Folge, dass sich auch die freiwerdenden Energien infolge einer unbeabsichtigten aber nicht völlig auszuschließenden Explosion des leicht entzündlichen Sauerstoff-Kraftstoffgemisches entsprechend verringern.
Die gleiche Temperaturerhöhung würde bei der Standardmethode dieses Risiko erheblich erhöhen. Versuche haben gezeigt, dass diese Energie über die dafür vorgeschriebene Berstscheibe nur relativ langsam abgeführt wird und auch aufgrund des damit austretenden Feuerstrahls das Bedienpersonals gefährden kann. Da die beschriebene beschleunigte Methode jedoch automatisch abläuft, kann hier auf Anwesenheit während der gesamten Heizperiode verzichtet werden. Das Hantieren erfolgt erst nach dem Abkühlprozess. Bei der Standard-Methode ist diese Anwesenheit zum Ein- und Ausbringen erforderlich.

### Ultraschall-Rührung:

Zur weiteren Beschleunigung des Oxidationsverhaltens bzw. besseren Durchmischung der Probe kann eine Beaufschlagung mit Ultraschall vorgesehen werden. Hierfür kann beispielsweise ein ultraschall-betriebener Schwinger am Boden oder direkt in der Probe vorgesehen werden.

Die Erfindung wird nachfolgend unter Bezugnahme auf die Figuren der Zeichnungen an Ausführungsbeispielen näher erläutert. Es zeigen:
- Fig. 1 a, b: Wiedergabe und Veranschaulichung einer Ausführungsform des erfindungsgemäßen Druckbehälters,
- Fig. 2: Diagramm zur Erläuterung der Brechpunkterfassung,
- Fig. 3: schematische Darstellung des Aufbaus und der Wirkungsweise der erfindungsgemäßen Vorrichtung,
- Fig. 4: Veranschaulichung der passiven Oberfläche des Druckbehälters.

Im Folgenden soll die Erfindung beispielhaft in größerem Detail beschrieben werden. Beispielhaft wird die Erfindung an dem Ausführungsbeispiel der Oxidationsbestimmung von Kraftstoff erläutert. Die Erfindung ist jedoch nicht darauf eingeschränkt, sondern an Stelle von Kraftstoff können auch andere Mineralölprodukte mit dem erfindungsgemäßen Verfahren getestet werden.

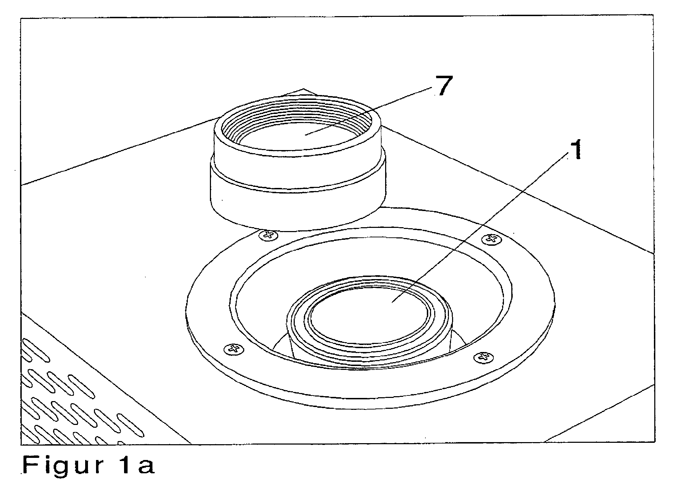

### Geräteaufbau (vgl. Figur 1 a, b):

Wie aus Figur 1b ersichtlich ist, besteht ein beispielhafter Druckbehälter 1 nach der Erfindung aus einem Schraubdeckel 7 mit Dichtung 8, welcher den Druckbehälter 1 dicht verschließen. Am Boden des Druckbehälters 1 ist die (temperaturgeregelte) Heizung 6 angeordnet. Alternativ kann die Heizung 6 auch in der beispielhaften Kraftstoffprobe 2 angeordnet sein. Die Temperatur wird durch einen Temperatur-Sensor 9 überwacht.

Zur Verhinderung chemisch/physikalisch/katalytischer Effekte zwischen Kraftstoffprobe 2 und Druckbehälter 1, die auftreten können, wenn die Kraftstoffprobe 2 ohne Glasgefäß direkt in den Druckbehälter 1 gegeben wird, wird die Innenwand des Druckbehälters 1 mit einer resistenten Oberfläche 11 versehen. Die Wandung 12 des Druckbehälters 1 kann aus einem nichtresistenten Material, beispielsweise Aluminium, bestehen.

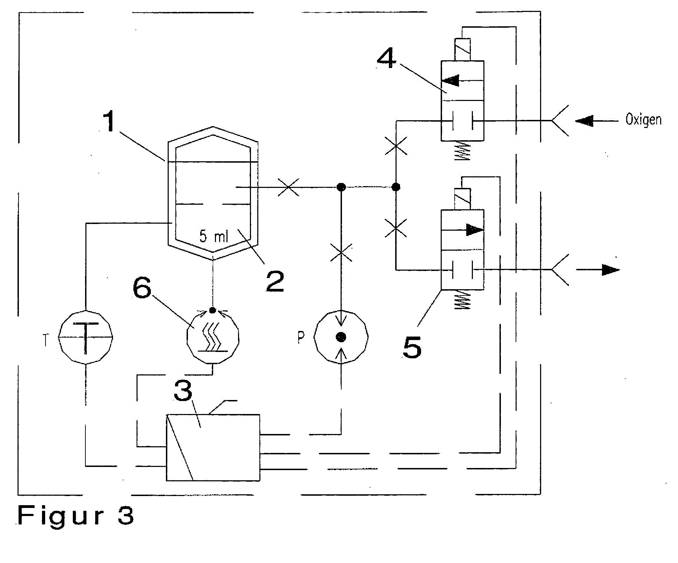

### Beschreibung des Programm-Ablaufes (vgl. Figur 3):

Der Druckbehälter 1 (Reaction Vessel) wird nach dessen Reinigung mit 5ml der Kraftstoffprobe 2 befüllt (Pipette, ggf. durch automatische Beschickung).
Der Deckel des Druckbehälters 1 wird gasdicht zugeschraubt. Das Programm wird durch eine Kontrolleinheit 3 (Control- and Operating Unit) verzögert gestartet, sodass der Anwender Gelegenheit hat, sich vom Gerät zu entfernen.

Die Kontrolleinheit 3 öffnet das Einlassventil 4 (Filling Valve) bis der Drucksensor P den Fülldruck von 500 kPa registriert.
Die Kontrolleinheit 3 öffnet das Auslassventil 5 (Purging) bis der Drucksensor P den Umgebungsdruck registriert.
Die Kontrolleinheit 3 öffnet das Einlassventil 4 (Filling Valve) bis der Drucksensor P den Fülldruck von 500 kPa registriert.

Die Heizung 6 (Heater) wird von der Kontrolleinheit 3 solange betrieben, bis der Temperatursensor T die Vorwahltemperatur (140°C) meldet.
Gleichzeitig wird die Zeit- und Druckmessung/-Registrierung gestartet.
Nach Erreichen der Vorwahltemperatur z.B. 140°C wird der maximal aufgetretene Druck als Pmax registriert.
Wenn der Druck unter Pmax-10% (Brechpunkt gefunden) gefallen ist, wird ein Gebläse zur Rückkühlung eingeschaltet.
Ist eine gewünschte Temperatur in der Nähe der Raumtemperatur unterschritten, wird zunächst der Restdruck abgelassen und anschließend dem Anwender signalisiert, so dass er den Druckbehälter 1 zum Reinigen öffnen kann.

Die Erfindung beschränkt sich in ihrer Ausführungsform nicht auf die vorstehend angegebenen bevorzugten Ausführungsbeispiele. Vielmehr ist eine Anzahl von Varianten denkbar, die von der erfindungsgemäßen Anordnung und dem erfindungsgemäßen Verfahren im Rahmen der Patentansprüche Gebrauch machen.

### Bezugszeichenliste

- 1: Druckbehälter
- 2: Probe, Kraftstoffprobe
- 3: Kontrolleinheit
- 4: Einlassventil
- 5: Auslassventil
- 6: Heizung
- 7: Schraubdeckel
- 8: Dichtung
- 9: Temperatur-Sensor
- 11: Resistente Oberfläche
- 12: Wandung des Druckbehälters

## Patentansprüche

1. Verfahren für eine beschleunigte Oxidationsbestimmung von Kraftstoffen oder Mineralölprodukten, wobei eine Probe (2) eines Kraftstoff- oder Mineralölproduktes in einen Druckbehälter (1) eingebracht und der Druck in dem Druckbehälter (1) überwacht wird, um die Oxidation zu bestimmen,
**dadurch gekennzeichnet, dass**
die Probe (2) direkt in den Druckbehälter (1) eingebracht und durch zumindest ein im Druckbehälter (1) und/oder in der Probe (2) angeordnetes Mittel zur Temperaturveränderung auf eine vorgebbare Temperatur über 100°C gebracht wird , wobei zur Festlegung eines Brechpunktes das Absinken des absoluten Druckwertes überwacht und der Brechpunkt als der Punkt festgesetzt wird, an dem der Druck um 10% von seinem Maximum abgesunken ist.

2. Verfahren nach Anspruch 1,
**dadurch gekennzeichnet, dass**
die Probe (2) auf eine Temperatur von 140°C gebracht wird.

3. Verfahren nach einem der Ansprüche 1 oder 2,
**dadurch gekennzeichnet, dass**
die vorgebbare Temperatur während der Oxidationsbestimmung konstant gehalten wird.

4. Verfahren nach einem der Ansprüche 1 bis 3,
**dadurch gekennzeichnet, dass**
eine Probe (2) von einem Volumen von 3 bis 20 ml eingebracht wird.

5. Verfahren nach einem der Ansprüche 1 bis 4,
**dadurch gekennzeichnet, dass**
Druckbehälter (1) und/oder Probe (2) mit Ultraschall beaufschlagt werden.

6. Vorrichtung für eine beschleunigte Oxidationsbestimmung von Kraftstoffen oder Mineralölprodukten, wobei die Vorrichtung einen Druckbehälter (1) zur Aufnahme von Proben (2) der Kraftstoffe oder Mineralölprodukte umfasst,
**dadurch gekennzeichnet, dass**
der Druckbehälter (1) zumindest ein Mittel zur Temperaturveränderung umfasst, wobei das zumindest eine Mittel zur Temperaturveränderung im Druckbehälter (1) und/oder in der Probe (2) angeordnet ist,
die Innenwand des Druckbehälters (1) wenigstens teilweise eine Oberfläche aufweist, die gegen chemische, physikalische und/oder katalytische Effekte bezüglich der Probe (2) resistent ist, und wobei
die Vorrichtung derart eingerichtet ist, dass folgendes ausführbar ist:
- Erhitzung der Probe (2) auf eine vorgebbare Temperatur über 100°C,
- Überwachung des Absinkens des absoluten Druckwertes, und
- Festsetzung eines Brechpunktes als des Punktes, an dem der Druck um 10% von seinem Maximum abgesunken ist.

7. Vorrichtung nach Anspruch 6,
**dadurch gekennzeichnet, dass**
das zumindest eine Mittel zur Temperaturveränderung als Heizung (6) ausgebildet ist.

8. Vorrichtung nach Anspruch 6 oder 7,
**dadurch gekennzeichnet, dass**
die resistente Oberfläche aus Gold ausgeführt ist.

9. Vorrichtung nach einem der Ansprüche 6 bis 8,
**dadurch gekennzeichnet, dass**
am Boden des Druckbehälters (1) und/oder in der Probe (2) ein ultraschall-betriebener Schwinger angeordnet ist.

10. Computerprogramm, das es einer Datenverarbeitungseinrichtung ermöglicht, nachdem es in Speichermittel der Datenverarbeitungseinrichtung geladen worden ist, ein Verfahren für eine beschleunigte Oxidationsbestimmung von Kraftstoffen oder Mineralölprodukten durchzuführen, wobei eine Probe (2) eines Kraftstoff- oder Mineralölproduktes direkt in einen Druckbehälter (1) eingebracht wird und die Probe (2) durch zumindest ein im Druckbehälter (1) und/oder in der Probe (2) angeordnetes Mittel zur Temperaturveränderung auf eine vorgebbare Temperatur über 100°C gebracht und der Druck in dem Druckbehälter (1) überwacht wird, um die Oxidation zu bestimmen, wobei zur Festlegung eines Brechpunktes das Absinken des absoluten Druckwertes überwacht und der Brechpunkt als der Punkt festgesetzt wird, an dem der Druck um 10% von seinem Maximum abgesunken ist.

11. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren für eine beschleunigte Oxidationsbestimmung von Kraftstoffen oder Mineralölprodukten durchzuführen, wobei eine Probe (2) eines Kraftstoff- oder Mineralölproduktes direkt in einen Druckbehälter (1) eingebracht wird und die Probe (2) durch zumindest ein im Druckbehälter (1) und/oder in der Probe (2) angeordnetes Mittel zur Temperaturveränderung auf eine vorgebbare Temperatur über 100°C gebracht und der Druck in dem Druckbehälter (1) überwacht wird, um die Oxidation zu bestimmen, wobei zur Festlegung eines Brechpunktes das Absinken des absoluten Druckwertes überwacht und der Brechpunkt als der Punkt festgesetzt wird, an dem der Druck um 10% von seinem Maximum abgesunken ist.

12. Computerlesbares Speichermedium, auf dem ein Computerprogramm nach Anspruch 10 gespeichert ist.

13. Verfahren, bei dem ein Computerprogramm nach Anspruch 10 aus einem elektronischen Datennetz, wie beispielsweise aus dem Internet, auf eine an das Datennetz angeschlossene Datenverarbeitungseinrichtung heruntergeladen wird.

## Claims

1. A method for accelerated determination of the oxidation of fuels or mineral oil products, wherein a sample (2) of a fuel or mineral oil product is introduced in a pressure vessel (1) and the pressure in the pressure vessel (1) is monitored in order to determine the oxidation,
**characterized in that**
the sample (2) is directly introduced into the pressure vessel (1) and heated to a predefinable temperature above 100°C by at least one means for changing temperature which is arranged in the pressure vessel (1) and/or in the sample (2) while, in order to define a breakpoint, the decrease of the absolute pressure value is monitored and the breakpoint is defined as the point where the pressure has decreased by 10% from its maximum value.

2. The method according to claim 1,
**characterized in that** the sample (2) is heated to a temperature of 140°C.

3. The method according to either of claims 1 or 2,
**characterized in that**
the predefinable temperature is kept constant while the oxidation is determined.

4. The method according to any one of claims 1 to 3,
**characterized in that**
a sample (2) of 3 to 20 ml in volume is introduced.

5. The method according to any one of claims 1 to 4,
**characterized in that**
the pressure vessel (1) and/or the sample (2) is/are exposed to ultrasound.

6. A device for accelerated determination of the oxidation of fuels or mineral oil products, wherein the device comprises a pressure vessel (1) for receiving samples (2) of the fuels or mineral oil products,
**characterized in that**
the pressure vessel (1) comprises at least one means for changing temperature, wherein the at least one means for changing temperature is arranged in the pressure vessel (1) and/or in the sample (2),
at least part of the inner wall of the pressure vessel (1) has a surface which is resistant to chemical, physical and/or catalytic effects relating to the sample (2), and wherein the device is configured to allow
- heating of the sample (2) to a predefinable temperature above 100°C,
- monitoring the decrease of the absolute pressure value, and
- defining a breakpoint as the point where the pressure has decreased by 10% from its maximum value.

7. The device according to claim 6,
**characterized in that**
the at least one means for changing temperature is designed as a heating unit (6).

8. The device according to claim 6 or 7,
**characterized in that**
the resistant surface is made of gold.

9. The device according to any one of claims 6 to 8,
**characterized in that**
an ultrasonic vibrator is arranged at the bottom of the pressure vessel (1) and/or in the sample (2).

10. A computer program which, once it has been loaded into storage means of a data processing device, enables said data processing device to carry out a method for accelerated determination of the oxidation of fuels or mineral oil products, wherein a sample (2) of a fuel or mineral oil product is directly introduced into a pressure vessel (1) and said sample (2) is heated to a predefinable temperature above 100°C by at least one means for changing temperature which is arranged in the pressure vessel (1) and/or in the sample (2) while the pressure in the pressure vessel (1) is monitored in order to determine the oxidation, wherein, in order to define a breakpoint, the decrease of the absolute pressure value is monitored and the breakpoint is defined as the point where the pressure has decreased by 10% from its maximum value.

11. A computer-readable storage medium where a program is stored which, once it has been loaded into storage means of a data processing device, enables said data processing device to carry out a method for accelerated determination of the oxidation of fuels or mineral oil products, wherein a sample (2) of a fuel or mineral oil product is directly introduced into a pressure vessel (1) and said sample (2) is heated to a predefinable temperature above 100°C by at least one means for changing temperature which is arranged in the pressure vessel (1) and/or in the sample (2) while the pressure in the pressure vessel (1) is monitored in order to determine the oxidation, wherein, in order to define a breakpoint, the decrease of the absolute pressure value is monitored and the breakpoint is defined as the point where the pressure has decreased by 10% from its maximum value.

12. A computer-readable storage medium where a computer program according to claim 10 is stored.

13. A method in which a computer program according to claim 10 is downloaded from an electronic data network, such as the internet, to a data processing device which is connected to said data network.

## Revendications

1. Procédé destiné à la détermination accélérée de l'oxydation de carburants ou de produits d'huile minérale, un échantillon (2) d'un produit de carburant ou d'huile minérale étant mis en place dans un réservoir sous pression (1) et la pression dans le réservoir sous pression (1) étant surveillée pour déterminer l'oxydation,
**caractérisé en ce que**
l'échantillon (2) est mis en place directement dans le réservoir sous pression (1) et est amené à une température paramétrable supérieure à 100 °C par au moins un moyen de modification de la température disposé dans le réservoir sous pression (1) et/ou dans l'échantillon (2),
la diminution de la valeur de pression absolue étant surveillée pour définir un point de fragilité, et le point de fragilité étant fixé comme le point auquel la pression a diminué de 10 % par rapport à son maximum,

2. Procédé selon la revendication 1,
**caractérisé en ce que**
l'échantillon (2) est amené à une température de 140 °C.

3. Procédé selon une des revendications 1 ou 2,
**caractérisé en ce que**
la température paramétrable est maintenue constante pendant la détermination de l'oxydation.

4. Procédé selon une des revendications 1 à 3,
**caractérisé en ce**
**qu'**un échantillon (2) ayant un volume de 3 à 20 ml est mis en place.

5. Procédé selon une des revendications 1 à 4,
**caractérisé en ce que**
le réservoir sous pression (1) et/ou l'échantillon (2) sont exposés à des ultrasons.

6. Dispositif destiné à la détermination accélérée de l'oxydation de carburants ou de produits d'huile minérale, le dispositif comprenant un réservoir sous pression (1) destiné à recevoir des échantillons (2) des carburants ou des produits d'huile minérale, **caractérisé en ce que**
le réservoir sous pression (1) comprend au moins un moyen de modification de la température, le moyen au moins au nombre de un de modification de la température étant disposé dans le réservoir sous pression (1) et/ou dans l'échantillon (2),
la paroi intérieure du réservoir sous pression (1) présente au moins partiellement une surface qui est résistante aux effets chimiques, physiques et/ou catalytiques en ce qui concerne l'échantillon (2), et
le dispositif étant agencé de telle sorte que les actions suivantes peuvent être réalisées :
- chauffage de l'échantillon (2) à une température paramétrable supérieure à 100 °C,
- surveillance de la diminution de la valeur de pression absolue,
- fixation d'un point de fragilité en tant que point auquel la pression a diminué de 10 % par rapport à son maximum.

7. Dispositif selon la revendication 6,
**caractérisé en ce que**
le moyen, au moins au nombre de un, de modification de la température est constitué en tant que chauffage (6).

8. Dispositif selon la revendication 6 ou 7,
**caractérisé en ce que**
la surface résistante est réalisée en or.

9. Dispositif selon une des revendications 6 à 8,
**caractérisé en ce**
**qu'**un oscillateur fonctionnant aux ultrasons est disposé sur le fond du réservoir sous pression (1) et/ou dans l'échantillon (2).

10. Programme informatique qui, après avoir été chargé dans des moyens de mémoire du dispositif de traitement de données, permet à un dispositif de traitement de données d'exécuter un procédé de détermination accélérée de l'oxydation de carburants ou de produits d'huile minérale, un échantillon (2) d'un produit de carburant ou d'huile minérale étant mis en place directement dans un réservoir sous pression (1) et l'échantillon (2) étant amené à une température paramétrable supérieure à 100 °C par au moins un moyen de modification de la température disposé dans le réservoir sous pression (1) et/ou dans l'échantillon (2), et la pression dans le réservoir sous pression (1) étant surveillée pour déterminer l'oxydation, la diminution de la valeur de pression absolue étant surveillée pour définir un point de fragilité, et le point de fragilité étant fixé comme le point auquel la pression a diminué de 10 % par rapport à son maximum.

11. Support de mémoire lisible par ordinateur, sur lequel est enregistré programme qui, après avoir été chargé dans des moyens de mémoire du dispositif de traitement de données, permet à un dispositif de traitement de données d'exécuter un procédé pour une détermination accélérée de l'oxydation de carburants ou de produits d'huile minérale, un échantillon (2) d'un produit de carburant ou d'huile minérale étant mis en place directement dans un réservoir sous pression (1) et l'échantillon (2) étant amené à une température paramétrable supérieure à 100 °C par au moins un moyen de modification de la température disposé dans le réservoir sous pression (1) et/ou dans l'échantillon (2), et la pression dans le réservoir sous pression (1) étant surveillée pour déterminer l'oxydation, la diminution de la valeur de pression absolue étant surveillée pour définir un point de fragilité, et le point de fragilité étant fixé comme le point auquel la pression a diminué de 10 % par rapport à son maximum.

12. Moyen de mémoire lisible par un ordinateur, sur lequel est enregistré un programme informatique selon la revendication 10.

13. Procédé dans lequel un programme informatique selon la revendication 10 est, à partir d'un réseau de donnée électronique, comme par exemple à partir d'Internet, téléchargé sur un dispositif de traitement de données raccordé au réseau de données.
